(19) Europäisches Patentamt
European Patent Office
Office européen des brevets

(11) Publication number: **0 342 150 A1**

(12) **EUROPEAN PATENT APPLICATION**

(21) Application number: **89730118.0**

(22) Date of filing: **10.05.89**

(51) Int. Cl.[4]: **C 07 D 285/12**
C 07 D 271/10,
C 07 D 261/10,
C 07 D 263/46, A 01 N 43/82,
A 01 N 43/76, A 01 N 43/80

(30) Priority: **13.05.88 DE 3816807**
**13.06.88 DE 3820456**
**15.06.88 DE 3820628**
**19.07.88 DE 3824879**

(43) Date of publication of application:
**15.11.89 Bulletin 89/46**

(84) Designated Contracting States:
**AT BE CH DE ES FR GB GR IT LI LU NL SE**

(71) Applicant: **SCHERING AKTIENGESELLSCHAFT Berlin und Bergkamen**
**Müllerstrasse 170/178 Postfach 65 03 11**
**D-1000 Berlin 65 (DE)**

(72) Inventor: **Hübl, Dieter, Dr.**
**Massmannstrasse 9**
**D-1000 Berlin 41 (DE)**

**Bühmann, Ulrich, Dr.**
**Am Volkspark 83**
**D-1000 Berlin 31 (DE)**

**Pieroh, Ernst**
**Im Fischgrund 18**
**D-1000 Berlin 28 (DE)**

**Joppien, Hartmut, Dr.**
**Juttastrasse 18**
**D-1000 Berlin 37 (DE)**

(54) **Substituted azole ethers, their preparation and use as pesticides.**

(57) The invention relates to new azole ethers of general formula I
A-SR[1] (I)
in which
A is the group,

a) 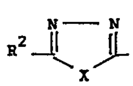 b) 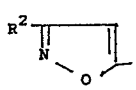 or c) 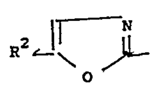

in which $R^1$ and $R^2$ have the meanings given in the description, processes for their preparation and their use as pesticides, especially plant pathogenic nematodes.

EP 0 342 150 A1

**Description**

## SUBSTITUTED AZOLE ETHERS, THEIR PREPARATION AND USE AS PESTICIDES

This invention relates to new substituted azole ethers, their preparation and use as pesticides, especially against nematodes.

Compounds of similar structure with nematicidal activity are already known, such as for example EP 217,747 and 263,066 and US 3,781,438.

The known compounds however are not usually sufficiently active or do not have sufficient plant compatibility.

It has now been found that azole ethers of general formula I

$A\text{-}SR^1$ (I)

in which

A is the group,

a) 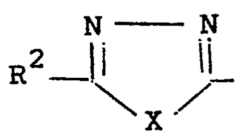 b) 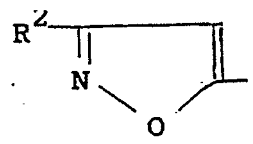 or c) 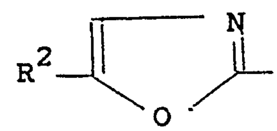

wherein

A) when A is the group a),

X is oxygen or sulphur and

either

(i) $R^1$ is $-CF_2Br$, $-CH_2\text{-}CF_3$, $-CF_2\text{-}CF_2Br$, $-CH_2\text{-}CF_2\text{-}CF_2H$ or $-CH_2\text{-}CH_2F$, and

$R^2$ is phenyl, biphenyl or naphthyl, each of which is optionally substituted, one or more times by the same or different halogen, $C_{1-4}$-alkyl, $C_{1-4}$-alkoxy, $C_{1-4}$-alkylthio, halo-$C_{1-4}$-alkyl, halo-$C_{1-4}$-alkoxy, halo-$C_{1-4}$-alkylthio, nitro or cyano, or

(ii) $R^1$ is $C_{1-12}$-alkyl or $C_{2-12}$-alkenyl, each of which is optionally substituted, one or more times by the same or different halogen and

$R^2$ is the group

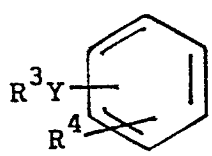

wherein

Y is oxygen or sulphur,

$R^3$ is fluoro-$C_{1-12}$-alkyl or fluoro-$C_{2-12}$-alkenyl, and

$R^4$ is hydrogen, halogen, $C_{1-6}$-alkyl,$C_{1-6}$-alkoxy, $C_{1-4}$-alkylthio, halo-$C_{1-4}$-alkyl, halo-$C_{1-4}$-alkoxy, halo-$C_{1-4}$-alkylthio or nitro or cyano, and

B) when A is the group b or c),

$R^1$ is $C_{1-12}$-alkyl or $C_{2-12}$-alkenyl, each of which is optionally substituted, one or more times by the same or different halogen, and

$R^2$ is phenyl, biphenyl or naphthyl, each of which is optionally substituted, one or more times by the same or different halogen, $C_{1-4}$-alkyl, $C_{1-4}$-alkoxy, $C_{1-4}$-alkylthio, halo-$C_{1-4}$-alkyl, halo-$C_{1-4}$-alkoxy, halo-$C_{1-4}$-alkylthio, nitro or cyano, show a surprisingly good nematicidal activity coupled with good plant compatibility.

The compounds of the invention also show good activity against biting and sucking insects and their eggs and also mites.

The term "haloalkyl" in relationship that one or more hydrogen atoms of the akyl group are replaced by halogen.

A preferred group of compounds are those where A is the group (a) and

(i) $R^1$ is bromodifluoromethyl and

$R^2$ is phenyl, optionally substituted (preferably is the 4-position) by halo, $C_{1-4}$-alkoxy or $C_{1-4}$-alkyl, or

(ii) $R^1$ is fluoro-$C_{1-4}$-alkyl or fluoro-$C_{2-4}$-alkenyl, optionally substituted by bromo, and

$R^3$ is phenyl, substituted (preferably in the 4-position) by fluoro-$C_{1-4}$-alkoxy.

In the case of (ii) $R^1$ is preferably bromodifluoromethyl or difluoromethyl.

The compounds of the invention of general formula I can be prepared according to known methods by reacting a compound general formula II,

$A\text{--}SY$ (II)

in which A has the meaning given above and Y is hydrogen, ammonium or an alkali metal, with a compound of

formula Z-$R^1$, in which Z is a leaving group such as for example halogen, mesylate and tosylate and $R^1$ has the meaning given above, in an inert solvent or solvent mixture, preferably at raised temperature and preferably at raised pressure, in the presence of a base.

Suitable bases include organic and inorganic bases, such as for example tertiary amines, eg triethylamine or tripropylamine, alkali metal and alkaline earth metal hydride, hydroxide, carbonate and bicarbonate and also alkali metal alcoholate, such as sodium ethylate or potassium tert.butylate.

Suitable solvents for the preparation of the compounds of the invention include for example diethyl ether, dioxane and tetrahydrofuran; aliphatic and aromatic hydrocarbons, such as toluene and petroleum ether; halogenated hydrocarbons, eg chlorobenzene, methylene chloride, carbon tetrachloride and chloroform; nitriles, such as for example acetonitrile and propionitrile; N,N-dialkylamides, such as for example dimethylformamide; ketons, eg acetone and methyl ethyl ketone; dimethyl sulphoxide, sulpholane, as well as water and alcohols, eg methanol, ethanol, isopropanol or butanol, and mixtures of such solvents.

Suitable catalysts are for example 18-crown-6, 15-crown-5 and tetraalkylammonium salts, such as tetra-n-butylammonium bromide.

The temperature of the reaction of the compounds of formula II to compounds of formula I depends of the reactants and can vary between -70 and 120°C. The pressure also depends on the reactants and can lie between 1 and 25 bar. The reaction usually lasts from ca. 0.5 to 48 hours. The reaction mixture can be poured into ice/water, extracted and worked up in known manner. The resulting products can be purified in conventional manner, for example by recrystallisation, vacuum distillation or column chromatography.

The compounds of formula II used as starting material are either known or can be obtained in an analogous way to known processes.

Because of the nematicidal activity coupled with good plant compatibility, the compound according to the invention can be successfully applied in plant protection as pesticides in agriculture, in vine and fruit growing, in horticulture and in forestry.

Plant parasitic nematodes which can be controlled according to the invention include for example root-knot nematodes, such as Meloidogyne incognita, Meloidogyne hapla and Meloidogyne javanica, cyst forming nematodes, such as Globodera rostochiensis, Heterodera schacktii, Heterodera avanae, Heterodera glycines and Heterodera trifolii, and stem and leaf eelworms, such as Ditylenchus dipsaci, Ditylenchus destructor, Aphelenchoides ritzemabosi, Pratylenchus neglectus, Pratylenchus penetrans, Pratylenchus curvitatus, as well as Tylenchorhynchus dudius, Tylenchorhynchus claytoni, Rotylenchus robustus, Heliocotylenchus multicinctus, Radopholus similis, Benlonolaimus longicaudatus, Longidorus elongatus and Trichodorus primitivus.

Many of the compounds of formula I and II also have insecticidal activity for example and as a result can be used for combating a wide range of insectes and acarids, including animal ectoparasites. Examples include Lepidoptera, such as Plutella xylostella, Spodoptera littoralis, Heliothis armigera and Pieris brassicae; Diptera, such as Musca domestica, Ceratitis capitata, Erioischia brassicae, Lucilia sericata and Aedes aegypti; Homoptera, including aphids such as Megoura viciae and Nilaparvata lugens; Coleoptera, such as Phaedon cochleariae, Anthonomus grandis, Epilachna varivestis and corn rootworms (Diabrotica spp. egDiabrotica undecimpunctata); Orthoptera, such as Blattella germanica; ticks, such as Boophilus microplus and lice, such as Damalinia bovis and Linognathus vituli, as well as spider mites such as Tetranychus urticae and Panonychus ulmi.

Based on the insecticidal, nematicidal and acaricidal properties of the invention, a wide range of pests can combated in various stages of plants as well as pests, including nematodes, in human ans animals.

The active ingredients of the invention can be used in the form of their commercial formulations and/or in the form of preparations obtained from these formulations.

The content of active ingredient used in the preparations prepared from the commercial formulations can vary over wide ranges. The rate of use of combating per hectare lies between about 0.03 kg and about 10 kg, preferably about 0,3 kg to about 6 kg.

The active ingredient can be applied in the usual formulations such as solutions, emulsions, wettable powders, suspensions, powders, dusts, foams, pastes, soluble powders, granules, aerosols, suspension concentrates, seed dressings, natural and synthetic substances impregnated with the active ingredients, microcapsules in polymers and in seed coatings for seeds, as well as formulations with burning substances such as smoke cartridges, smoke capsules and smoke spirals amongst others as well as ULV-cold and hot fogging formulations.

These formulations can be prepared in known manner for example by mixing the active ingredient with diluents such as liquid solvents, and liquefied gases and/or solid carriers, optionally using surface active agents such as emulsifiers and/or dispersing agents and/or forming agents.

When using water as the diluent, organic solvents can also be used for example as auxiliary solvents.

Examples of liquid solvents include aromatic hydrocarbons, such as xylene, toluene or alkynaphthalene, chlorinated aromatic or chlorinated aliphatic hydrocarbons, such as chlorobenzene, chloroethylene or methylene dichloride, aliphatic hydrocarbons, such as cychlohexane or paraffins, for example mineral oil fractions, alcohols, such as butanol and glycol as well as their ethers and esters, ketones, such as acetone, methyl ethyl ketone, methyl isobutyl ketone or cyclohexanone, strongly polar solvents, such as dimethylformamide and dimethyl sulphoxide, as well as water.

By the term liquefied gaseous diluents or carriers are meant those substances which are gaseous at normal

temperature and pressure, for example aerosol blowing agents, such as halohydrocarbons, as well as butane, propane, nitrogen and carbon dioxide.

Example of solid carriers are natural earth powders, such as kaolin, alumina, talc, chalk, quartz, attapulgite, montmorillonite or diatomaceous earths and synthetic powders, such as finely divided silica, aluminium oxide and silicates as well as solid carriers for granules, crushed and fractionated natural rocks, such as calcite, marble, pumice, sepiolith and dolomite, as well as synthetic granules from inorganic and organic powders as well as granules from organic materials such as sawdust, coconut shells, maize cobs amd tobacco stalks.

Examples of emulsifying and/or foaming agents include non-ionic and anionic emulsifiers, such as polyoxyethylene-fatty acid esters, polyoxyethylene-fatty alcohol ethers, for example alkylaryl-polyglycolethers, alkylsulphonates and arylsulphonates as well as protein hydrolysates.

Dispersing agents include for example lignin, sulphite waste liquors and methylcellulose.

There can also be used in the formulations sticking agents such as carboxymethylcellulose, natural and synthetic powdery, granulated or latex-forming polymers, as well as gum arabic, polyvinyl alcohol and polyvinyl acetate.

There can also be used dyestuffs, such as inorganic pigments, for example iron oxide, titanium oxide, ferrocyan blue and organic dyestuffs such as alizarin-and azo-metal phthalocyanine dyestuffs and trace elements such as salts of iron, manganese, boron, copper, cobalt, molybdenum and zinc.

The formulations contain in general between 0.1 and 95 weight percent of active ingredient, preferably between 0.5 and 90 percent.

Examples of formulations are:

I. Wettable powder

10 parts by weight of the compound of the invention are intimately mixed with 12 parts by weight of calcium lignosulphonate, 76 parts by weight of finely divided kaolin and 2 parts by weight of dialkylnaphthalene sulphonate and then milled.

II. Dusting powder

2.5 parts by weight of the compound of the invention were dissolved in 10 methylene chloride and added to a mixture of 20 parts by weight of finely divided silicic acid and 71.5 parts by weight talc and 1 part by weight Sudan red. The solvent was removed in vacuo and the residue finely milled.

III. Granule

5 parts by weight of by weight of the compound of the invention were dissolved in 10 parts by weight of methylene chloride and sprayed onto 95 parts by weight granulated attapulgite of particle size 0.3 mm - 0.8 mm and dried.

IV. Emulsifiable concentrate

20 parts by weight of the compound of the invention were dissolved in a mixture of 75 parts by weight of isophorone and 5 parts by weight of a mixture of 30 parts by weight of calcium benzene sulphonate and 30 parts by weight of castor oil polyglycolate with 40 mole % ethylene oxide and 40 parts by weight of a copolymer of propylene- and ethylene oxide.

Novel starting materials were prepared as follows

(5-(4-Difluoromethoxyphenyl)-1,3,4-oxadiazole-2-thiol

14.0g (0.069 mol) 4-Difluoromethoxbenzhydrazide were dissolved in 300 ml ethanol and treated with 3.87 g (0.069 mol) potassium hydroxide dissolved in 17 ml water. 7.6 g (0.10 mol) Carbon disulphide was added dropwise, with stirring, at room temperature. The mixture was heated under reflux for 7 hours. It was then concentrated to dryness, the residue taken up in ca. 500 ml water and filtered. The clear solution was treated, with stirring, with concentrated sulphuric acid until it was pH 2. The resulting crystals were separated and washed with water.

Yield : 14.1 g = 83% of theory

m.p.: 155-157° C

5-(4-Difluoromethoxyphenyl)-1,3,4-thiadiazole-2-thiol

9.7 g (0.031 mol) Potassium 4-difluoromethoxbenzoyl carbazate was added portionwise at 5° C to 45 ml concentrated sulphuric acid. The mixture was stirred for for 30 minutes at 0° C, then added to 300 ml water and the resulting precipitate separated and washed three times with water.

Yield: 6.9 g = 83.5% of theory m.p.: 178-179° C

In a similar manner the following compounds of formula I, in which A is

a) 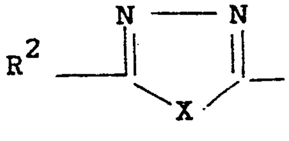

$R^1$ is hydrogen and X and $R^2$ are as shown below.

$R^2$ = 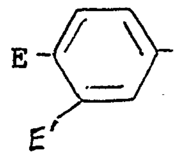

| No | E | E' | X | mp (°C) |
|---|---|---|---|---|
| 3 | $-OCH_2CF_3$ | H | O | 192 |
| 4 | $-OCH_2CF_3$ | H | S | 214 |
| 5 | $-OCF_2CHF_2$ | H | O | * |
| 6 | $-OCF_2CHF_2$ | H | S | * |
| 7 | $-OCF_3$ | H | O | * |
| 8 | $-OCF_3$ | H | S | * |
| 9 | $-OCH_2CF_2CHF_2$ | H | O | 110 |
| 10 | $-OCH_2CF_2CHF_2$ | H | S | 136 |
| 11 | H | $-OCF_2H$ | O | 166-172 |
| 12 | H | $-OCF_2H$ | S | 180-183 |

* = unpurified starting material

The following Examples illustrate the preparation of compounds of the invention and their use.

Example 1

2-Bromodifluoromethylthio-5-(4-chlorophenyl)-1,3,4-thiadiazole

1.65 g (0.069 mol) of an 80% suspension of sodium hydride in paraffin oil, which had been washed with 10 ml toluene, was suspended in 20 ml dimethylformamide. At a temperature of 5-10°C, a solution of 5.5 g (0.024 g) 5-(4-chlorophenyl)-1,3,4-thiadiazole-2-thiol in 40 ml dimethylformamide was added dropwise and the mixture stirred for 30 minutes at 10°C. 11.6g (0.055 mol) Dibromodifluoromethane was added dropwise and the mixture stirred for 3 hours at 10°C. The mixture was added to 400 ml ice/water and extracted three times with 100 ml ethyl acetate. The combined organic phases were washed twice with water, dried over magnesium sulphate and concentrated . The residue was purified by column chromatography (eluent: hexane/ ethyl acetete 4:1).

Yield: 2.40 g = 13.2% of theory

m.p.: 72°C

Example 2

5-(4-Difluoromethoxyphenyl)-2-bromodifluoromethylthio-1,3,4-thiadiazole

10 g (0.038 mol) 5-(4-difluoromethoxyphenyl)-1,3,4-thiadiazole-2-thiol was suspended in 50 ml dioxane, and, under vigorous stirring, 31 g of aqueous 34% potassium hydroxide was added dropwise. The mixture was heated to 60-70°C and whilst being vigorously stirred, a slow stream of chlorodifluoromethane was passed through. The mixture was added to 300 ml ice/water and extracted three times with 100 ml ethyl acetate. The combined organic phase was dried over magnesium sulphate and concentrated . The residue was purified by column chromatography (eluent: hexane/ ethyl acetete 4:1).

Yield: 2.83 g = 22.8% of theory

$n_D^{20}$: 1.5754

In a similar manner the following compounds of the invention of formula I were obtained, in which A is

a) 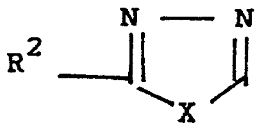

and X, $R^1$ and $R^2$ are as shown below.

$R^2$ = 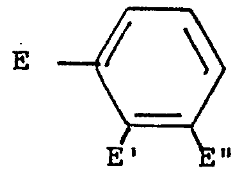

| No | E | E' | E" | X | $R^1$ | mp(°C)/$n_D^{20}$ |
|---|---|---|---|---|---|---|
| 3 | $-OCHF_2$ | H | H | O | $-CHF_2$ | 1.5278 |
| 4 | $-OCHF_2$ | H | H | O | $-CF{=}CFCF_3$ | |
| 5 | $-OCHF_2$ | H | H | S | $-CF{=}CFCF_3$ | |
| 6 | $-OCHF_2$ | H | H | O | $-CF_3$ | 1.51086 |
| 7 | $-OCHF_2$ | H | H | S | $-CF_3$ | |
| 8 | $-OCHF_2$ | H | H | O | $-CF_2Br$ | 23 |
| 9 | $-OCHF_2$ | H | H | S | $-CF_2Br$ | |
| 10 | $-OCHF_2$ | H | H | S | $-CF_2CF_2Br$ | |
| 11 | $-OCH_2CF_3$ | H | H | S | $-CF_2Br$ | 89 |
| 12 | $-OCH_2CF_3$ | H | H | O | $-CHF_2$ | 77 |
| 13 | $-OCH_2CF_3$ | H | H | S | $-CHF_2$ | 111 |
| 14 | $-OCH_2CF_3$ | H | H | O | $-CF_2Br$ | 69 |
| 15 | $-OCH_2CF_3$ | H | H | O | $-CF_3$ | 87 |
| 16 | $-OCH_2CF_3$ | H | H | S | $-CF_3$ | 117 |
| 17 | $-OCHF_2$ | H | H | O | $-CH_2CH_2CF{=}CF_2$ | 48 |
| 18 | $-OCH_2CF_2CHF_2$ | H | H | O | $-CHF_2$ | 62 |
| 19 | $-OCH_2CF_2CHF_2$ | H | H | S | $-CHF_2$ | 74 |
| 20 | $-OCH_2CF_2CHF_2$ | H | H | O | $-CF_2Br$ | 1.53082 |
| 21 | $-OCH_2CF_2CHF_2$ | H | H | S | $-CF_2Br$ | 76 |
| 22 | $-OCF_2Br$ | H | H | S | $-CF_2Br$ | 51 |
| 23 | H | $-OCHF_2$ | H | O | $-CF_2Br$ | 1.54696 |
| 24 | H | $-OCHF_2$ | H | O | $-CHF_2$ | 1.52982 |

| No | E | E' | E" | X | $R^1$ | mp(°C)/$n_D^{20}$ |
|---|---|---|---|---|---|---|
| 25 | H | $-OCHF_2$ | H | O | $-CH_2CH_2CH_2CF_3$ | 1.50838 |
| 26 | H | $-OCHF_2$ | H | S | $-CHF_2$ | 1.57826 |
| 27 | $-OCH_2CHF_3$ | H | H | O | $-CH_2CH_2CF{=}CF_2$ | 77.6 |
| 28 | $-OCH_2CHF_3$ | H | H | S | $-CH_2CH_2CF{=}CF_2$ | |
| 29 | $-OCF_3$ | H | H | O | $-CHF_2$ | |
| 30 | $-OCF_3$ | H | H | S | $-CHF_2$ | |
| 31 | $-OCF_3$ | H | H | O | $-CF_2Br$ | |
| 32 | $-OCF_3$ | H | H | S | $-CF_2Br$ | |
| 33 | $-OCF_2Br$ | H | H | O | $-CF_2Br$ | Oil |
| 34 | $-OCHF_2$ | H | H | S | $-CH_2CH_2CF{=}CF_2$ | |
| 35 | Cl | H | H | O | $-CF_2Br$ | 73 |
| 36 | Cl | H | H | O | $-CH_2CF_3$ | 90 |
| 37 | H | H | Cl | O | $-CF_2Br$ | 48 |
| 38 | F | H | H | O | $-CF_2Br$ | 54.5 |
| 39 | $CH_3O-$ | H | H | O | $-CF_2Br$ | 93 |
| 40 | F | H | H | S | $-CF_2Br$ | 60 |
| 41 | $CH_3O-$ | H | H | S | $-CF_2Br$ | 94 |
| 42 | H | H | Cl | S | $-CF_2Br$ | 73.8 |
| 43 | H | Cl | H | S | $-CF_2Br$ | 63.8 |
| 44 | H | H | F | S | $-CF_2Br$ | 93 |
| 45 | H | H | F | O | $-CF_2Br$ | 71.5 |
| 46 | H | Cl | H | O | $-CF_2Br$ | 52.7 |
| 47 | $CH_3$ | H | H | S | $-CF_2Br$ | 93 |
| 48 | Cl | H | H | O | $-CF_2CF_2Br$ | 1.55998 |
| 49 | $CH_3$ | H | H | O | $-CF_2Br$ | 105 |
| 50 | Cl | H | H | S | $-CF_2CF_2Br$ | 59 |
| 51 | Cl | H | Cl | O | $-CF_2Br$ | 1.60328 |
| 52 | Cl | H | Cl | S | $-CF_2Br$ | 63 |
| 53 | H | H | H | O | $-CF_2Br$ | 79 |
| 54 | H | H | H | S | $-CF_2Br$ | 70 |
| 55 | H | $-CF_3$ | H | O | $-CF_2Br$ | Oil |

Example 56

5-Difluoromethylthio-3-phenylisoxazole

7 g (0.032 mol) Potassium 3-phenylisoxazole-5-thiolate was suspended in 22 ml dioxane and 20 g of 25% aqueous potassium hydroxide added dropwise. The mixture was heated to 60-70°C and a slow stream of

chlorodifluoromethane was passed through over 3 hours. The mixture was added to 300 ml ice/water and extracted three times with 100 ml ethyl acetate. The combined organic phases were dried over magnesium sulphate and concentrated.
Yield: 5.6 g = 80.6% of theory
$n_D^{20}$: 1.5608

Example 57 and 58

5-(1,2,2,2-tetrafluoromethyl-1-trifluoromethylethylthio)-3-phenylisoxazole (No 57)

5-(2,2-Difluoromethyl-1-trifluoromethylvinylthio)-3-phenylisoxazole (No 58)

6.4 g (0.03 mol) Potassium 3-phenylisoxazole-5-thiolate was dissolved in 60 ml dimethylformamide in an autoclave. 26 g (0.017 mol) 1,1,2,3,3,3-hexafluoroprop-1-ene was added under pressure (3.5 bar) and the mixture heated at 70°C over 25 hours. After cooling and depressurisation the solution was added to 300 ml ice/water and extracted three times with 100 ml ethyl acetate. The combined organic phases were dried over magnesium sulphate and concentrated. The reulting products were separated and purified by column chromatography.
Yield (No 57): 2.35 g = 22.7% of theory/oil
Yield (No 58): 2.65 g = 28.8% of theory/mp: 35°C.

In a similar manner the following compounds of the invention of formula I were obtained, in which A is

b) 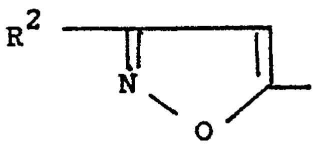

and $R^1$ and $R^2$ are as shown below.

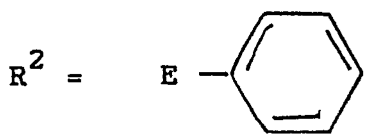

| No | E | $R^1$ | mp(°C) |
|---|---|---|---|
| 59 | Cl | $-CHF_2$ | 40-42 |
| 60 | Cl | $-CF_2Br$ | 55-56 |
| 61 | Cl | $-CF_2CF_2Br$ | 30-32 |
| 62 | H | 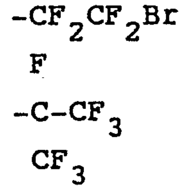 | 55 |
| 63 | F | $-CHF_2$ | 68 |
| 64 | Cl | $-CH_2CH_2CF=CF_2$ | 43 |
| 65 | H | $-CH_2CH_2CF=CF_2$ | |
| 66 | $F_2HCO$ | $-CHF_2$ | Oil |

Example 67

2-Bromodifluoromethylthio-5-(4-chlorophenyl)oxazole

2.14 g (0.07 mol) of a 80% suspension of sodium hydride in paraffin oil, which had been washed once with 10 ml toluene, was suspended in 15 ml dimethylformamide. At a temperature of 20-30°C, a solution of 7.50 g (0.035 mol) 5-(4-chlorophenyl)-2-oxazolethiol in 100 ml dimethylformamide was added dropwise and the mixture stirred for 30 minutes at room temperature. 7.34 g (0.035 mol) Dibromodifluoromethane was added dropwise and the mixture stirred for 12 hours at room temperature. The mixture was added to 300 ml ice/water and extracted three times with 100 ml ethyl acetate. The combined organic phases were dried over magnesium sulphate and concentrated. The residue was purified by column chromatography (eluent: hexane/ ethyl acetete 4:1).
Yield: 5.93 g = 49.7% of theory
$n_D^{20}$: 1.6313

Example 68

(5-(4-Chlorophenyl)-2-difluoromethylthiooxazole

A solution of 5 g (0.024 mol) 5-(4-chlorophenyl)-2-oxazolethiol in 22 ml dioxane was treated with 9.6 g (0.24 mol) sodium hydroxide in 22 ml water and the mixture was stirred and heated to 70°C. At this temperature, a uniform stream of chlorodifluoromethane was passed through over 60 minutes. The mixture was added to 1 l ice/water, made weakly acid and extracted several times with dichloromethane. The organic phase was dried over magnesium sulphate, filtered and concentrated. The residual oil was chromatographed on silica gel.
Yield: 5.16 g = 82.2% of theory
$n_D^{20}$: 1.5997

Example 69

(5-(4-Chlorophenyl)-2-(2,2,2-trifluoroethylthio)oxazole

A solution of 10 g (O.048 mol) (5-(4-chlorophenyl)-2-oxazolethiol in 50 ml dimethylformamide was slowly added dropwise to a suspension of 1.44 g (0.048 mol) of 80% of sodium hydride in 50 ml dimethylformamide, which had been cooled to 0°C. After hydrogen evolution had ceased, 6.1 g (0.024 mol) 2,2,2-trifluoroethyl tosylate in 20 ml dimethylformamide was slowly added dropwise. The mixture was heated at 110°C under nitrogen for 6 hours. After cooling, it was added to 1000 ml ice/water and extracted several times with ether. The ether phase was washed with water and dried over magnesium sulphate and concentrated. The residual oil was chromatographed on silica gel.
Yield: 3.0 g = 42.5% of theory
m.p.: 92-94°C

In a similar manner the following compounds of the invention of formula I were obtained, in which A is

c) 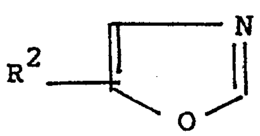

and $R^1$ and $R^2$ are as shown below.

$R^2$ = 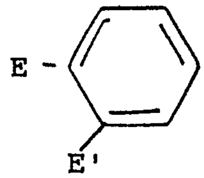

| No | E | E' | $R^1$ | mp(°C)/$n_D^{20}$ |
|---|---|---|---|---|
| 70 | H | H | $-CHF_2$ | 1.58316 |
| 71 | Cl | Cl | $-CHF_2$ | 1.6082 |
| 72 | Cl | H | $-CF=CF-CF_3$ * <br> $-C(CF_3)=CF_2$ | 1.59874 |
| 73 | $F_2HCO-$ | H | $-CF_2Br$ | 1.56478 |
| 74 | $F_2HCO-$ | H | $-CHF_2$ | 65 |
| 75 | F | H | $-CF_2Br$ | 40 |
| 76 | F | H | $-CHF_2$ | 35 |
| 77 | Cl | H | $-CH_2CH_2CF=CF_2$ | 1.57942 |
| 78 | F | H | $-CH_2CH_2CF=CF_2$ | 1.54430 |

* = 1:1 mixture

Use Example A

Control of root knot nematode (Meloidogyne incognita)

The composition of the invention was mixed thoroughly in the form of a 10% powder preparation with soil that had been strongly infested with the test nematode. After this the treated soil was put into a 0.5 litre fermenting tube, treated wth cucumber seeds and cultivated at a soil temperature of 25-27° C in a greenhouse. After a cultivation time of 25 to 28 days, the cucumber roots were washed and inspected in a water bath for nematode attack (root knots) and the % level of activity of the active ingredients compared with a treated control was determined. When the nematode attack is fully controlled the level of activity is 100%.

At a dose of 25 mg of active substance per litre of soil, a nematode attack by Meloidogyne incognita was fully controlled (100%) by the compounds of Examples 1, 2, 5, 8, 12, 14-18, 23, 24, 26, 35, 38-41, 43-47, 49, 53, 54, 56, 59, 63, 64, 68, 70 and 74-76.

Use Example B

Activity in the curative treatment of field beans (Phaseolus vulgaris nanus Aschers.) against motile stages of the two spotted mite (Tetranychus urticae Koch)

In a heated greenhouse, seedlings of field beans were grown to full development of the primary leaf and then covered with bits of leaf infested with Tetranychus urticae. One day later the leaf bits were removed and the plants sprayed with 0.1% aqueous preparation of the active ingredient until dripping wet. After 7 days at 22 to 24° C, the amount of dead motile stages of Tetranychus on treated and untreated plants was ascertained. The activity was calculated according to Abbott.

For the compounds of the invention of Example 2, 35, 55, 59 and 68 this amounted to 80% or more.

Use Example C

Activity in the curative treatment of field beans (Phaseolus vulgaris nanus Aschers.) against eggs of the two spotted mite (Tetranychus urticae Koch)

In a heated greenhouse, seedlings of field beans were grown to full development of the primary leaf and then treated with adult females of Tetranychus urticae. One day later the plants, carrying the eggs which had benn laid i9n the meantime, were sprayed with 0.1% aqueous preparation of the active ingredient until dripping wet. After 7 days at 22 to 24° C, the amount of dead motile stage of Tetranychus on treated and untreated plants was ascertained. The activity was calculated according to Abbott.

Compound of the invention of Examples 2, 37, 38, 42, 56, 61, 68, 70 and 74 showed a greater than 80% activity.

Use Example D

Activity in prophylactic treatment of leaves against brown rice-hoppers (Nilarparvata lugens Stal).

In a heated greenhouse, rice seedlings (about 15 per pot) were grown until formation of the third leaf and then sprayed until dripping wet with an aqueous preparation containing 0.1% of active material. After drying the sprayed leaves, a transparent cylinder was placed over each pot. 30 Adult brown rice-hoppers (Nilarparvata lugens) were introduced into each pot. After 2 days at 26° C in the greenhouse, the amount of dead hoppers was determined. The activity was calculated according to Abbott in comparison with several untreated control pots.

The compounds of Examples 3, 11-18, 20, 26, 35-38, 40-46, 56, 59-62, 64, 67, 68, 70 and 72-74 showed 100% activity.

Use Example E

Activity against eggs/larvae of the corn rootworm (Diabrotica undecimpunctata)

Formulations of compounds of the invention were made up as aqueous emulsions at a concentration of 0.1%. These preparations were sprayed onto soil in polystyrene petri dishes each of which contained 1 maize seedling and about 50 eggs of the corn rootworm (Diabrotica undecimpunctata) at arate of 4 mg/$cm^2$. The closed pots were left in the glasshouse under extended daylight conditions for 4 days. The criterion for judging the activity was the mortality eggs or the newly emerged larvae.

The compounds of Examples 1-3, 23, 24, 35, 37, 38, 40-42, 48-51, 53, 54, 56 and 72-75 showed 80-100% activity.

Use Example F

Ovicidal activity against eggs of the cotton army worm (Spodoptera littoralis).

The compounds of the invention were made up as aqueous suspensions or emulsions at a concentration of 0.1%. One day old eggs that had been laid on filter paper by fertilized female moths were dipped in the preparations until they were completely wet and then placed in closed petri dishes in the laboratory under extended daylight conditions for four days. The % inhibition of hatching of the eggs in comparison with untreated eggs indicates the level of activity.

The compounds of Examples 2, 3, 35, 56, 68, 69, 73 and 74 showed 80-100% activity.

Use Example G

Activity in the prophylactic treatment of feed against the two spotted mite (Tetranychus urticae Koch)

From the primary leaf of field beans (Phaseolus vulgaris nanus Aschers.) 14 mm diameter discs were cut. Some of these were treated with a 0.1% aqueous preparations of compounds of the invention and these along side untreated discs were placed on filter papers with the underside of the leaves turned upwards. After drying the test pieces, they were each infested with 7 adult female Tetranychus urticae and maintained for 3 days at 25° C and 16 hours light per day. The experiment was replicated 4 times. Dead and alive adults were then counted and removed. Similarly the number of eggs laid were counted. After a further 7 days, the number of living larvae were counted. The activity was calculated using Abbott's method in comparison with the untreated controls.

The compounds of Examples 1, 11-18, 20 ,21, 23, 25, 26, 38, 40-48, 50-54, 59-62, 64 and 72-74 showed 80-100% activity.

Use Example H

Ovicidal activity against egg masses of the cotton bollworm (Heliothis viriscens)

Formulations of compounds of the invention were made up with an active ingredient content of 0.1%. One day old eggs that had been laid on filter paper by fertilised female moths were dipped in the preparations until they were completely wet and then placed in closed petri dishes in the laboratory under extended daylight conditions for four days. The % inhibition of hatching of the eggs in comparison with untreated eggs indicates the level of activity.

The compounds of Examples 2, 3, 14, 23, 24, 26, 38, 42-44, 48, 50-54, 56, 68, 73 and 74 showed 80-100% activity.

Use Example I

Activity in the prophylactic treatment of feed against the black bean aphids (Aphis fabae scop.)

From the primary leaf of field beans (Phaseolus vulgaris nanus Aschers.) 24 mm diameter discs were cut. Some of these were treated with a 0.1% aqueous preparations of compounds of the invention and these along side untreated discs were placed on filter papers with the underside of the leaves turned upwards. After drying the test pieces, they were each infested with wingless stages of Aphis fabae (aprox. 100/leaf disc). The experiment was replicated 3 times. The leaf discs on the wet filter paper were maintained for 2 days at 25°C and 16 hours light per day. The activity was calculated using Abbott's method in comparison with the untreated controls.

The compounds of Examples 23, 61 and 74 showed more than 80% activity.

Use Example J

Activity against larvae (L 2) of the cotton bollworm (Heliothis viriscens)

Formulations of compounds of the invention were made up with an active ingredient content of 0.1%. Into these pieces of feedstuff were dipped for 2 seconds each. After drying, the treated feed was placed in polystyrene petri dishes. One hour later, 10 larvae of the cotton bollworm (Heliothis viriscens) were counted into each dish. The closed dishes were maintained for up to 7 days at 25°C under extended daylight conditions. The criterion for judging the activity was the % mortality of the larvae at the end of the experiment.

The compounds of Examples 67, 68 and 75 showed 80-100% activity.

Use Example K

Insecticidal activity against Musca domestica

Aliquots of acetone solutions of test compounds at various concentrations were applied to 9 cm diameter filter papers placed in the bottom of 9 cm diameter petri dishes closed by glass lids. After evaporation of solvent, the treated surfaces, together with control treated with acetone alone, were then infested with adult houseflies, (Musca domestica) and held at 22°C for 24 hours. The percentage mortality of the inserts was then recorded.

Less than 5% mortality resulted in the control treatments whereas the compounds of Examples 35, 46, 56, 68 and 71 had an $LC_{50}$ of 1000 mg/$m^2$ or less.

Use Example L

Insecticidal activity against Lucilia sericata

1 ml aliquots of an acetone solution containing test compound at various concentrations were applied to cotton wool dental rolls 1 cm x 2 cm, contained in glass vials (2cm diameter x 5 cm long). After drying, the treated materials were then impregnated with 1ml of nutrient solution, infested with first instar larvae of sheep blowfly (Lucilia sericata), closed by a cotton wool plug and held at 25°C for 24 hours.

For the controls the mortality was <5% whereas the compounds of Examples 16, 17, 43, 44, 46, 56, 68, 71 and 77 had an $LC_{50}$ of 300 ppm or less.

Use Example M

Tickicidal activity against Boophilus microplus

9 cm diameter filter papers were impregnated with 1 ml aliquots of acetone solutions of test compound at various concentrations. The papers were allowed to dry and then folded into envelopes in which cattle tick larvae, (Boophilus microplus) were enclosed and held at 25°C and 80% R.H. for 48 hours. The percentage mortality of tick larvae was then recorded and compared with controls.

The controls gave a mortality of less than 5% whereas compounds of Examples 16, 35, 40, 42-44, 46, 56, 68 and 71 caused 50% mortality at a concentration of 300 ppm or less.

**Claims**

1. Azole ethers of general formula I
A-$SR^1$ (I)
in which
A is the group,

a) 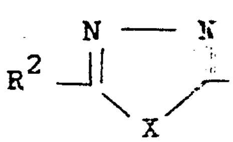 b) 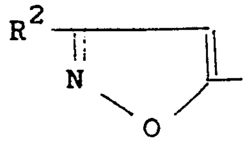 or c) 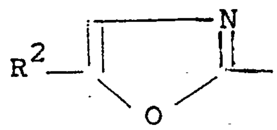

wherein

A) when A is the group a),

X is oxygen or sulphur and

either (i) $R^1$ is $-CF_2Br$, $-CH_2-CF_3$, $-CF_2-CF_2Br$, $-CH_2-CF_2-CF_2H$ or $-CH_2-CH_2F$, and

$R^2$ is phenyl, biphenyl or naphthyl, each of which is optionally substituted, one or more times by the same or different halogen, $C_{1-4}$-alkyl, $C_{1-4}$-alkoxy, $C_{1-4}$-alkylthio, halo-$C_{1-4}$-alkyl, halo-$C_{1-4}$-alkoxy, halo-$C_{1-4}$-alkylthio, nitro or cyano, or

(ii) $R^1$ is $C_{1-12}$-alkyl or $C_{2-12}$-alkenyl, each of which is optionally substituted, one or more times by the same or different halogen and

$R^2$ is the group

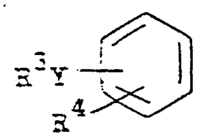

wherein

Y is oxygen or sulphur

$R^3$ is fluoro-$C_{1-12}$-alkyl or fluoro-$C_{2-12}$-alkenyl, and

$R^4$ is hydrogen, halogen, $C_{1-6}$-alkyl, $C_{1-6}$-alkoxy, $C_{1-4}$-alkylthio, halo-$C_{1-4}$-alkyl, halo-$C_{1-4}$-alkoxy, halo-$C_{1-4}$-alkylthio or nitro or cyano, and

B) when A is the group b or c),

$R^1$ is $C_{1-12}$-alkyl or $C_{2-12}$-alkenyl, each of which is optionally substituted, one or more times by the same or different halogen, and

$R^2$ is phenyl, biphenyl or naphthyl, each of which is optionally substituted, one or more times by the same or different halogen, $C_{1-4}$-alkyl, $C_{1-4}$-alkoxy, $C_{1-4}$-alkylthio, halo-$C_{1-4}$-alkyl, halo-$C_{1-4}$-alkoxy, halo-$C_{1-4}$-alkylthio, nitro or cyano.

2. Compounds according claim 1, wherein

A is the group (a) and

(i) $R^1$ is bromodifluoromethyl and

$R^2$ is phenyl, optionally substituted (preferably is the 4-position) by halo, $C_{1-4}$-alkoxy or $C_{1-4}$-alkyl, or

(ii) $R^1$ is fluoro-$C_{1-4}$-alkyl or fluoro-$C_{2-4}$-alkenyl, optionally substituted by bromo, and

$R^3$ is phenyl, substituted (preferably in the 4-position) by fluoro-$C_{1-4}$-alkoxy.

3. A pesticidal composition which comprises a compound according to claim 1 or 2, in admixture with agriculturally acceptable carriers or diluents.

4. A method of combating pests which comprises applying to the pests or their locus a compound according to claim 1 or 2.

European Patent Office

# EUROPEAN SEARCH REPORT

Application number

EP 89730118.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP - A2/A3 - 0 217 747 (CIBA-GEIGY AG) * Claims 1,3,11,12,17 * -- | 1,3,4 | C 07 D 285/12<br>C 07 D 271/10<br>C 07 D 261/10<br>C 07 D 263/46<br>A 01 N 43/82<br>A 01 N 43/76<br>A 01 N 43/80 |
| A | EP - A1 - 0 220 025 (SHIONOGI & CO., LTD.) * Claims 1,6,7,9 * -- | 1,3,4 | |
| A | US - A - 4 001 228 (MATTALIA) * Claim 1 * ---- | 1,3,4 | |

TECHNICAL FIELDS SEARCHED (Int. Cl.4)

C 07 D 285/00
C 07 D 271/00
C 07 D 261/00
C 07 D 263/00

The present search report has been drawn up for all claims

| Place of search | Date of completion of the search | Examiner |
|---|---|---|
| VIENNA | 05-07-1989 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82